**Europäisches Patentamt**

(19) **European Patent Office**

**Office européen des brevets**

(11) Publication number : **0 417 191 B1**

# EUROPEAN PATENT SPECIFICATION

(45) Date of publication of patent specification :
10.03.93 Bulletin 93/10

(51) Int. Cl.⁵ : **A61K 39/395, A61K 47/00**

(21) Application number : **89906915.7**

(22) Date of filing : **25.05.89**

(86) International application number :
**PCT/US89/02289**

(87) International publication number :
**WO 89/11298 30.11.89 Gazette 89/28**

(54) FORMULATION FOR ANTIBODY REAGENTS.

(30) Priority : **27.05.88 US 199936**

(43) Date of publication of application :
**20.03.91 Bulletin 91/12**

(45) Publication of the grant of the patent :
**10.03.93 Bulletin 93/10**

(84) Designated Contracting States :
**AT BE CH DE FR GB IT LI LU NL SE**

(56) References cited :
**EP-A- 0 073 371
EP-A- 0 124 018
CH-A- 645 537
US-A- 4 186 129**

(73) Proprietor : **CENTOCOR, INC.
244 Great Valley Parkway
Malvern, PA 19355 (US)**

(72) Inventor : **SHEALEY, David, J.
1322 Broadview West
Downingtown, PA 19335 (US)**
Inventor : **PHILLIPS, Christopher, P.
P.O. Box 65
Brandamore, PA 19316 (US)**

(74) Representative : **Harvey, David Gareth et al
Graham Watt & Co. Riverhead
Sevenoaks Kent TN13 2BN (GB)**

## Description

Background of the Invention

This invention relates generally to antibody preparations and particularly to a highly stabilized antibody preparation for parenteral administration.

It is well known that many protein preparations intended for administration to humans require stabilizers to prevent denaturation of the proteins, agglomeration and other alterations to the proteins prior to the use of the preparation. Many preparations are particularly unstable in dilute solutions. This instability is manifested in the formation of insoluble particles, and is often increased when the protein preparation is stored, or shipped. This phenomenon, known as "shedding", is often increased when the protein preparation is stored at room temperature or higher, so that the preparation must often be refrigerated. "Shedding" generally refers to a visible precipitation of protein molecules.

Various methods for stabilizing protein preparations have been used with varying degrees of success. For example, increasing the concentration of the protein or adding another protein such as human serum albumin (HSA) has been known to enhance stability in some cases. However, such preparations may not always be acceptable for therapeutic purposes. In considering an appropriate stabilizer, such factors as lack as antigenicity, the biological activity of the specific proteins being stabilized and the availability and cost of the stabilizer are important.

Various carbohydrates have been used to stabilize and/or enhance solubility of certain biologically active protein preparations. For example, U.S. Patent 4,186,192, to Lundblad et al. discloses the use of maltose to increase the stability of an immune serum globulin preparation for intramuscular or intravenous administration. In U.S. Patent 2,826,533, Fowell discloses the use of dextrose to increase the solubility of a fibrinogen preparation. In U.S. Patent 4,089,944 Thomas discloses the use of a variety of carbohydrates, such as dextrose, mannose, galactose, fructose, lactose, sucrose, and maltose to increase the solubility of an AHF-fibrinogen composition. It has been found that when dextrose is added to immune serum globulin to enhance stability and or solubility, the globulins tend to aggregate over time, thereby increasing the optical density of the solution, and resulting in shedding.

The exact nature of shedding is not fully understood. Shedding is an undesirable manifestation since it is visually observable and indicates the possibility that the shedded protein may be inactive or denatured, therefore reducing the effective amount of protein available. A protein preparation in which shedding is apparent is an unsatisfactory product in terms of visual appearance.

The formation of protein aggregates and particulates has long been considered a problem in the development of parenteral immunoglobulin products. The administration of immunoglobulin G, for example, was limited to the intramuscular route because of endogenous anticomplementary activity due to aggregated immunoglobulin until the recent development of chemically and enzymatically treated immunoglobulin G. J. E. Pennington, Rev.Inf.Dis., 8(4):5371-5373 (1986). Recent modifications in immunoglobulin G formulations have also helped to alleviate this problem. J. P. McCue et al., Rev. Inf.Dis., 8(4):5374-5381 (1986). However, most commercially available formulations now in use require filtration of the product prior to injection to remove these aggregates and particulates.

The addition of maltose to protein solutions for various purposes is known. Maltose is readily available in pure form and has good stability in aqueous solutions in concentrations up to 20% by weight. Preparations containing maltose can be autoclaved without browning of the solution. Maltose in small quantities is practically physiologically inert. When administered parenterally, it is partially converted to glucose by the specific enzyme maltase found in many tissue sites in most animal species, including humans. The conversion to glucose is gradual and frequently undetectable when plasma glucose is serially measured; therefore there is no apparent increase in circulating insulin levels. Since maltose is a disaccharide, a 10% solution is approximately isotonic in humans. In U.S. Patent 4,499,073, Tenold discloses the use of a carbohydrate in a preparation of immune serum globulin to impart physiologically acceptable isotonicity to the preparation. Tenold specifies 10% weight to volume maltose for this purpose. Lundblad et al., report in U.S. Patent 4,186,192 that a solution of immune serum globulin is stabilized when maltose is added in a concentration of between 5 and 18% by weight. Fernandes et al. describe a preparation of intravenous gamma-globulin stabilized with maltose to minimize precipitation and improve in vitro shelf stability. Vox Sang,39:101-112(1980).

Summary of the Invention

The invention comprises an aqueous stabilizing buffer containing an antibody or antibody fragments, and maltose. This buffer composition has the ability to inhibit the antibody or antibody fragments in solutions intended for intravenous administration from precipitating and forming particulates in the final product vial. The antibody or antibody fragment may be derivatized with a chelating agent, such as, for example, diethylenetriamiepentaacetic acid (DTPA) for binding radiometals. The formulation of the buffer solution contains phosphate, sodium chloride and maltose. The present composition has been successful in stabilizing monoclonal antibodies, or fragments thereof, for shipping and short-term storage at ambient temperatures without loss of immunoreactivity, and requires no refrigeration or other special handling. The invention provides a stable, liquid formulation for monoclonal antibody products without shedding, thus increasing the shelf life of the antibody product.

## Description of The Preferred Embodiments

The aqueous stabilizing buffer of this invention minimizes the formation of protein aggregates and particulates in reagents containing antibodies or antibody fragments, and insures that the antibody in solution maintains its immunoreactivity over time. The preparation comprises a sterile, pharmaceutically acceptable solution containing a phosphate buffer, sodium chloride, an antimyosin monoclonal antibody or antibody fragment, and maltose. A preferred embodiment of this invention comprises about 10mM to about 100mM sodium phosphate (pH 6-8), about 145mM sodium chloride and about 5-20% (w/v) maltose and between about 0.5-5.2 mg/ml antibody, preferably antimyosin. However, other antibodies or fragments, for example, antifibrin may be used. This buffer enhances the stability of immunological activity of the monoclonal antibody, and prevents the immunoglobulins in solution intended for intravenous administration to human subjects from precipitating and forming particulates in the final product vial. Another embodiment of the formulation contains pure monoclonal antibody molecules, or fragments, that have been modified for diagnostic therapeutic applications; for example derivatized with a chelating agent such as diethylenetriamine° pentaacetic acid (DTPA). The derivatized antibody can then be used as a radiopharmaceutical due to the chelator's ability to bind a radioactive heavy metal, such as, for example, Indium-111. In a more preferred embodiment, the antibody solution includes a monoclonal antibody fragment, such as antimyosin, derivatized with DTPA. The chelating agent is used for incorporating a radiometal, such as Indiun-111, into the antibody protein, forming a protein-chelate-radiometal complex. This complex is then administered to a subject to deliver the radiometal to a site defined by the antigen which is the target of the antibody. The radiolabeled antibody can be used in scintigraphy, for example, in the imaging of tumors, or of disease sites, such as mycocardial infarct or blood clots. For example, injection of labeled antimyosin antibody, which is specific for cardiac myosin, will result in localization of the radiometal at the site where antimyosin binds to myosin, and the site can then be scanned with a gamma camera to obtain an image of the myocardium useful for diagnostic purposes.

The present formulation exhibits superior stabilizing characteristics in terms of minimal protein particle formation, preservation of immunoreactivity and radiometal incorporation over time, and under stress conditions, such as elevated temperatures, vial filling and shipping.

Maltose, which is used to stabilize the antibody solution, is described in detail in, for example, the Merck Index, 10th edition, Merck and Co., Inc. Rahway, NJ (1983). Maltose is a disaccharide, (4-0-α-D-glucopyranosyl-D-glucopyranose), which has been established as useful for maintaining pharmaceutically acceptable isotonicity of immunoglobulin solutions. (See U.S. Patent 4,499,073 to Tenold and U.S. Patent 4,186,192 to Lundblad et al., both discussed hereinabove). It has also been determined that maltose is not metabolized by humans when administered intravenously, and is excreted as maltose, with no apparent elevation in blood glucose levels or release of insulin.

Buffers have long been used to solubilize and stabilize antibody products for parenteral injection. They are utilized as biologically acceptable carriers for proteins. Protein solubility in the buffer solution depends upon a number of factors, such as ionic strength of the solution and the isoelectric point of the protein. Buffers which have been used as antibody carriers include citrate, sodium chloride and phosphate. The preferred buffer for this formulation is sodium phosphate buffers.

Sodium chloride is added to antibody compositions to enhance stability and to render the solution physiologically acceptable upon injection. Other alkali metal salts, such as potassium chloride, are not physiologically acceptable when injected intraveneously.

Stability studies have demonstrated that a composition of the invention has successfully maintained the following characteristics after 65 weeks: antibody solubility (determined via liquid borne particulate analysis), chelator activity (greater than 88% binding of Indium-111 at 10 minutes), antibody immunoactivity (when compared to reference standard material) and antibody molecular integrity (via high pressure liquid chromatography and SDS page electrophoresis comparisons to reference standard material.

The invention is further illustrated by the following examples:

Example 1

Preparation of an Optimized Antimyosin Fab-DTPA Formulation

Formulations were tested with a variety of buffers, salt concentrations, pH levels, and excipients, such as, human serum albumin, surfactants, mandelic acid and N-acetyl tryptophanate. The test formulations were initially screened by visual inspection after incubation at 4°C, 22°C, 37°C and 45°C.

MATERIALS AND METHODS

Protein Samples

a. Antimyosin Fab-DTPA (Centocor, Inc., lot # 00745), 0.5 mg/ml in 100 mM sodium citrate, pH 5.0; manufactured 3/15/85. Source of antibody: Ascites fluid.
b. Antimyosin Fab-DTPA (Centocor, Inc., lot # 03505), 5.2 mg/ml in 0.9% NaCl, manufactured 12/16/85. Source of antibody: Cell supernatant

Buffers Tested

The following reagents were used to make the test buffers:
Sodium citrate (Sigma Chemical Co., St. Louis, MO)
Sodium chloride (J.T. Baker Co.)
Sodium phosphate Monobasic and Dibasic (Sigma Chemical Co.)
Maltose (Sigma Chemical Co.)
Lactose (Sigma Chemical Co.)
Tween® 80 (Sigma Chemical Co.)
Dextrose (Sigma Chemical Co.)
Human Serum Albumin (25%) (Armour)
Propylene Glycol (Fisher Scientific Co.)
Sodium acetate (Sigma Chemical Co.)
Trishydroxymethylaminomethane (tris buffer) (Sigma Chemical Co.)
Hydroxyethyl piperazine ethane sulfonic acid (HEPES buffer) (Sigma Chemical Co.)
N-acetyl tryptophanate (Sigma Chemical Co.)
Mandelic acid (Sigma Chemical Co.)

Test buffers:

1. 100 mM Na Citrate Buffer, pH 5
2. 100 mM Citrate Buffer, 0.05% Tween® 20, pH 5
3. 100 mM Citrate Buffer, 0.01% Tween® 20, pH 5
4. 100 mM Na Citrate Buffer, 0.2% Tween® 20, pH 5
5. 100 mM Na Citrate Buffer, 5% Lactose, pH 5
6. 100 mM Na Citrate Buffer, 7.5% Lactose, pH 5
7. 100 mM Na Citrate Buffer, 10% Lactose, pH 5
8. 100 mM Na Citrate Buffer, 5% Dextrose, pH 5
9. 100 mM Na Citrate Buffer, 7.5% Dextrose, pH 5
10. 100 mM Na Citrate Buffer, 10% Dextrose, pH 5
11. 100 mM Na Citrate Buffer, 5% Maltose, pH 5
12. 100 mM Na Citrate Buffer, 7.5% Maltose, pH 5
13. 100 mM Na Citrate Buffer, 10% Maltose, pH 5
14. 100 mM Na Citrate Buffer, 0.5% Human Serum Albumin (HSA), pH 5
15. 100 mM Na Citrate Buffer, 1% HSA, pH 5
16. 100 mM Na Citrate Buffer, 2% HSA, pH 5
17. 100 mM Na Citrate Buffer, pH 5.5
18. 100 mM Na Citrate Buffer, 5% Maltose, pH 5.5
19. 100 mM Na Citrate Buffer, 7.5% Maltose, pH 5.5
20. 100 mM Na Citrate Buffer, 10% Maltose, pH 5.5
21. 100 mM Na Citrate Buffer, 0.5% HSA, pH 5.5

22. 100 mM Na Citrate Buffer, 1% HSA, pH 5.5
23. 100 mM Na Citrate Buffer, 2% HSA, pH 5.5
24. 100 mM Na Citrate Buffer, pH 6
25. 100 mM Na Citrate Buffer, 5% Maltose, pH 6
26. 100 mM Na Citrate Buffer, 7.5% Maltose, pH 6
27. 100 mM Na Citrate Buffer, 10% Maltose, pH 6
28. 100 mM Na Citrate Buffer, 0.5% HSA, pH 6
29. 100 mM Na Citrate Buffer, 1% HSA, pH 6
30. 100 mM Na Citrate Buffer, 2% HSA, pH 6
31. 100 mM Na Citrate Buffer, 100 mM Sodium Chloride, pH 5
32. 100 mM Na Citrate Buffer, 200 mM Sodium Chloride, pH 5
33. 100 mM Na Citrate Buffer, 0.025% Tween® 20, pH 5
34. 100 mM Na Citrate Buffer, 0.01% Tween® 20, pH 5
35. 100 mM Na Citrate Buffer, 0.05% Tween® 80, pH 5
36. 100 mM Na Citrate Buffer, 0.025% Tween® 80, pH 5
37. 100 mM Na Citrate Buffer, 0.01% Tween® 80, pH 5
38. 100 mM Na Citrate Buffer, 0.1% Propylene Glycol, pH 5
39. 100 mM Na Citrate Buffer, 0.05% Propylene Glycol, pH 5
40. 100 mM Na Citrate Buffer, 0.01% Propylene Glycol, pH 5
41. 500 mM Na Acetate Buffer, pH 5
42. 10 mM Phosphate Buffer, pH 7.4
43. 25 mM Phosphate Buffer, pH 7.3
44. 25 mM Tris Buffer, pH 7.3
45. 25 mM HEPES Buffer, pH 7.3
46. 25 mM Tris Buffer, 100 mM Sodium Chloride, pH 7.3
47. 25 mM HEPES Buffer, 100 mM Sodium Chloride, pH 7.3
48. 100 mM Phosphate Buffer, pH 7.4
49. 100 mM Phosphate Buffer, 100 mM Sodium Chloride, pH 7.4
50. 100 mM Citrate Buffer, 20 mM N-Acetyl Tryptophanate, pH 5
51. 100 mM Na Citrate Buffer, 20 mM Mandelic Acid, pH 5
52. 200 mM Na Citrate Buffer, 20 mM N-Acetyl Tryptophanate, 20 mM Mandelic Acid, pH 5
53. 100 mM Na Citrate Buffer, 100 mM Sodium Chloride, 20 mM N-Acetyl Tryptophanate, pH 5
54. 100 mM Na Citrate Buffer, 100 mM Sodium Chloride, 20 mM Mandelic Acid, pH 5
55. 100 mM Na Citrate Buffer, 100 mM Sodium Chloride, 20 mM Mandelic Acid, 20 mM N-Acetyl Tryptophanate, pH 5
56. 100 mM Na Citrate Buffer, 100 mM Sodium Chloride, 1% HSA, pH 5
57. 12.5 mM Phosphate Buffer, 100 mM Sodium Chloride, pH 7.2
58. 25 mM Phosphate Buffer, 100 mM Sodium Chloride, pH 7.2
59. 50 mM Phosphate Buffer, 100 mM Sodium Chloride, pH 7.2
60. 100 mM Phosphate Buffer, 100 mM Sodium Chloride, pH 7.2
61. 12.5 mM Phosphate Buffer, pH 7.2
62. 25 mM Phosphate Buffer, pH 7.2
63. 50 mM Phosphate Buffer, pH 7.2
64. 100 mM Phosphate Buffer, pH 7.2
65. 12.5 mM Phosphate Buffer 0.1% Tween® 80, pH 7.2
66. 25 mM Phosphate Buffer, 0.1% Tween® 80, pH 7.2
67. 50 mM Phosphate Buffer, 0.1% Tween® 80, pH 7.2
68. 100 mM Phosphate Buffer, 0.1% Tween® 80, pH 7.2
69. 12.5 mM Phosphate Buffer, 100 mM Sodium Chloride, 0.1% Tween 80, pH 7.2
70. 25 mM Phosphate Buffer, 100 mM Sodium Chloride 0.1% Tween 80, pH 7.2
71. 50 mM Phosphate Buffer, 100 mM Sodium Chloride, 0.1% Tween 80, pH 7.2
72. 100 mM Phosphate Buffer, 100 mM Sodium Chloride, 0.1% Tween 80, pH 7.2
73. 12.5 mM Phosphate Buffer, 0.1% Propylene Glycol, pH 7.2
74. 24 mM Phosphate Buffer, 0.1% Propylene Glycol, pH 7.2
75. 50 mM Phosphate Buffer, 0.1% Propylene Glycol. pH 7.2
76. 100 mM Phosphate Buffer, 0.1% Propylene Glycol, pH 7.2
77. 12.5 mM Phosphate Buffer, 100 mM Sodium Chloride, 0.1% Propylene Glycol, pH 7.2
78. 25 mM Phosphate Buffer, 100 mM Sodium Chloride, 0.1% Propylene Glycol, pH 7.2

79. 50 mM Phosphate Buffer, 100 mM Sodium Chloride, 0.1% Propylene Glycol, pH 7.2

80. 100 mM Phosphate Buffer, 100 mM Sodium Chloride, 0.1% Propylene Glycol, pH 7.2

81. 100 mM Na Citrate Buffer, 1% HSA, $8 \times 10^{-4}$M Mandelic Acid, pH 5

82. 100 mM Na Citrate Buffer, 100 mM Sodium Chloride, 1% HSA, $8 \times 10^{-4}$M Mandelic Acid, pH 5

83. 100 mM Na Citrate Buffer, 1% HSA, $8 \times 10^{-4}$M N-Acetyl Tryptophanate, pH 5

84. 100 mM Na Citrate Buffer, 1% HSA, 100 mM Sodium Chloride, $8 \times 10^{-4}$M N-Acetyl Tryptophanate, pH 5

85. 100 mM Na Citrate Buffer, 1% HSA, $8 \times 10^{-4}$M Mandelic Acid, $8 \times 10^{-4}$M N-Acetyl Tryptophanate, pH 5

86. 100 mM Na Citrate Buffer, 1% HSA, 100 mM Sodium Chloride, $8 \times 10^{-4}$M Mandelic Acid, $8 \times 10^{-4}$M N-Acetyl Trytophanate, pH 5

87. 100 mM Na Acetate Buffer, 1% HSA, 100 mM Sodium Chloride, 10% Maltose, pH 5

88. 100 mM Na Acetate Buffer, 100 mM Sodium Chloride, 10% Maltose, pH 5

89. 10 mM Phosphate Buffer, 145 mM Sodium Chloride, pH 7.2

90. 10 mM Phosphate Buffer, 145 mM Sodium Chloride, 10% Maltose, pH 7.2

91. 10 mM Phosphate Buffer, 145 mM Sodium Chloride, 10% Maltose, pH 7.0

92. 10 mM Phosphate Buffer, 145 mM Sodium Chloride, 10% Maltose, pH 6.75

93. 10 mM Phosphate Buffer, 145 mM Sodium Chloride, 10% Maltose, pH 6.5

94. 10 mM Phosphate Buffer, 145 mM Sodium Chloride, 10% Maltose, pH 6.25

95. 10 mM Phosphate Buffer, 145 mM Sodium Chloride, 10% Maltose, pH 6.0

## Formulation Adjustment and Testing

Antimyosin Fab-DTPA was dialyzed against 100 volumes of the indicated buffers using dialysis tubing (Fisher Scientific Co.), and then 0.2 μm filtered. Dialysis tubing was boiled in 10 mM EDTA pH 7.0, rinsed with distilled water, and stored in 70% ethanol prior to use.

Dialyzed antimyosin Fab-DTPA was adjusted to 0.5 mg/ml and filled, 1 ml/vial, aseptically into sterile 1 ml vials (Wheaton) and sealed with sterile rubber stoppers (West) and metal crimps. Vials were incubated at 4°C, 22°C, 37°C and 45°C for 48-96 hours. Some vials were, in addition, stressed by shaking at 37°C (formulations 1-32) or by shipping in styrofoam containers from Malvern, PA to Miami, FL, then returned to Malvern, PA by Federal Express, Priority 1 mail (formulations 90-95). Shipped vials were filled using a peristaltic pump (Paxall) through 0.123 inch (3.075 mm inside diameter) silicone tubing at a pump speed of 500 rpm.

Vials were visually inspected by inverting several times and observing against a dark and a light background. Vials containing antimyosin Fab-DTPA were compared with control vials, prepared as described above, which contained buffer only. In general, all of these buffers exhibited no shedding at 4°C and 22°C over the times they were examined. Experimental parameters for formulations exposed to temperatures of 37°C and 45°C yielded the most information. The degree of precipitation was graded as follows: + (precipitates, cloudy); +/- (fine precipitates); - (no precipitates). The results of the visual inspection for buffers 1-89 for formulations stressed at 37°C and 45°C are shown in Table 1.

## TABLE 1

Visual Inspection of Antimyosin Formulations
Stressed at 37°C and 45°C[a]

|            |       | 37°C Shaken (#1-#32) 45°C (#32-89) |             |       |       |
|------------|-------|-----------|-------------|-------|-------|
| Formulation | 37°C | | Formulation | 37°C | 45°C |
| 1  | −   | −   | 46 | +  | −  |
| 2  | +   | −   | 47 | −  | −  |
| 3  | −   | −   | 48 | −  | +  |
| 4  | −   | −   | 49 | −  | +  |
| 5  | +/− | +   | 50 | −  | +  |
| 6  | −   | +   | 51 | −  | +  |
| 7  | −   | +   | 52 | +  | +  |
| 8  | −   | +   | 53 | −  | +  |
| 9  | −   | +   | 54 | +  | +  |
| 10 | −   | +   | 55 | +  | +  |
| 11 | +/− | +   | 56 | −  | −  |
| 12 | −   | +   | 57 | +  | +  |
| 13 | −   | +   | 58 | +  | +  |
| 14 | −   | +   | 59 | +  | +  |
| 15 | −   | +   | 60 | +  | +  |
| 16 | −   | +   | 61 | −  | +  |
| 17 | −   | +   | 62 | +  | +  |
| 18 | −   | +   | 63 | +  | +  |
| 19 | +/− | +   | 64 | +  | +  |

7

| | | | | | |
|---|---|---|---|---|---|
| 20 | + | + | 65 | + | + |
| 21 | – | + | 66 | + | + |
| 22 | + | + | 67 | + | + |
| 23 | + | + | 68 | + | + |
| 24 | – | + | 69 | + | + |
| 25 | + | – | 70 | + | + |
| 26 | + | – | 71 | + | + |
| 27 | + | +/– | 72 | + | + |
| 28 | – | + | 73 | – | + |
| 29 | – | + | 74 | + | + |
| 30 | – | + | 75 | + | + |
| 31 | – | – | 76 | + | + |
| 32 | – | + | 77 | + | + |
| 33 | – | + | 78 | + | + |
| 34 | – | + | 79 | – | + |
| 35 | – | + | 80 | – | + |
| 36 | – | + | 81 | – | + |
| 37 | – | + | 82 | – | + |
| 38 | – | + | 83 | – | + |
| 39 | – | + | 84 | – | + |
| 40 | – | + | 85 | + | + |
| 41 | + | + | 86 | + | + |
| 42 | – | +/– | 87 | – | + |
| 43 | – | +/– | 88 | – | + |
| 44 | + | + | 89 | – | – |
| 45 | – | + | | | |

(a)  Incubated at indicated temperature 48-96 hr, or or shaken at maximum rpm on rotary shaker for 18 hr.

+   easily visible particulates, cloudy

+/–  fine particulates visible

–   no particulates

The phosphate formulations shown as formulations 90-95, were shipped to Miami, FL and returned to Malvern, PA, and formation of particulates was observed. The results of the shipping study are shown in Table 2.

## TABLE 2

### Shipping Study of Phosphate Formulations[a]

| Forumulation | pH | Particulates |
|---|---|---|
| 90 | 7.2 | + |
| 91 | 7.0 | +/- |
| 92 | 6.75 | - |
| 93 | 6.5 | - |
| 94 | 6.25 | - |
| 95 | 6.0 | - |

(a) 10 mM phosphate, 145 mM NaCl, 10% maltose containing 0.5 mg/ml antimyosin Fab-DTPA.

After storage at 4°C for over 2 years, the shipped vials were analyzed for particulates by particle counting in a Climet model CI-1000 particle counter. Each vial was degassed and three 0.1 ml aliquots counted to determine total particles greater than or equal to 10 µm and greater than or equal to 25 µm. Replicate runs were averaged and the total counts per dose (1.15 ml) are reported in Table 3.

## TABLE 3

### Particle Counts for Antimyosin Formulations[a]
### Shipped and Stored at 4°C for 25 Months

| Formulations | pH | total particles per dose | |
|---|---|---|---|
| | | 10 µm | 25 µm |
| 90 | 7.20 | 830 | 460 |
| 91 | 7.00 | not tested | not tested |
| 92 | 6.75 | 370 | 210 |
| 93 | 6.50 | 430 | 260 |
| 94 | 6.25 | 680 | 520 |
| 95 | 6.00 | 530 | 370 |
| specifications[b] | | 10,000 | 1,000 |

(a)  10 mM sodium phosphate, 145 mM sodium chloride, 10% maltose

(b)  USP XXI

These results confirm the results obtained by visual inspection of the shipped vials set out in Table 2. Protein precipitation during shipping or long term storage (more than 2 years) was minimized by the 10 mM sodium phosphate, 145 mM sodium chloride, 10% maltose formulation, particularly at pH 6.75 and 6.50.

In-111 Incorporation and Immunoreactivity

After screening buffers for particulate formation, selected formulations which showed minimal precipitation were further evaluated for In-111 incorporation into the protein, and immunoreactivity of the resulting In-111 labeled antimyosin Fab-DTPA. Formulations at neutral pH were acidified prior to radiolabeling with an equal volume of 0.2 M sodium citrate (pH 5) in a metal-free microfuge tube (BioRad). All transfers were also performed with metal-free pipette tips (BioRad). In-111 chloride [Amersham, 370 MBq/ml (10 mCi/ml) at reference] was then added to the protein-citrate mixtures to a final specific activity of 148 MBq (4 mCi) per milligram. After incubation for 15 minutes at room temperature, 10 ul was spotted 1.5 cm from one end of a 1 x 10 cm ITLC-SG paper strip (Gelman) and developed in 0.1 M sodium citrate (pH 5.0). The strip was cut in half and both halves measured in a dose calibrator set for In-111. Under these conditions, all In-111 that was protein bound remained at the origin.

In order to test for immunoreactivity, a 1000-fold dilution of the radiolabeled antimyosin Fab-DTPA was made in 0.01 M sodium phosphate (pH 7.2), 0.15 M NaCl, 1% (w/v) bovine serum albumin (PBS-1% BSA). One hundred microliters of this diluted sample were applied to a 1 ml column of myosin-Sepharose®C1-4B (Pharmacia). This affinity column was prepared by the attachment of myosin purified from dog heart tissue to cyanogen-bromide activated Sepharose® C1-4B (Pharmacia). The column was eluted with eight 1 ml aliquots of PBS-1% BSA, followed by eight 1 ml aliquots of 0.1M glycine pH 2,5, 0.01% thimerosal. The collected fractions were counted in a gamma counter set for In-111, the percentage eluting with the glycine buffer representing active radiolabeled antibody. This percentage was divided by the fraction of In-111 protein bound (from ITLC-SG chromatography) to correct for unbound In-111.

Those formulations showing the least amount of precipitation were examined to see if the excipients would adversely effect In-111 incorporation or activity of the antibody. Table 4 shows an example with the excipient human serum albumin, where it appears that a portion of the In-111 binds to sites on the albumin rather than to the antimyosin antibody.

## TABLE 4

### Comparison of Shipped Formulations With and Without Human Serum Albumin (HSA)

| Formulation | % Incorporation | % Immunoreactivity |
|---|---|---|
| 100 mM Citrate pH 5.0 (control) | 88.9 | 83.3 |
| 100 mM Citrate pH 5.0, 100 NaCl, 10% maltose | 96.5 | 71.8 |
| 100 mM Citrate pH 5.0, 100 mM NaCl, 10% maltose, 1% HSA | 92.4 | 55.2 |

It was discovered that the majority of excipients either did not prevent precipitation, or as in the case of HSA, interfered with In-111 binding to the antimyosin antibody. The best formulations were at neutral pH in HEPES [N-(2-hydroxyethyl)piperazine-N'-(2-ethanesulfonic acid)] or phosphate buffer.

Neutral pH formulations require acidification prior to In-111 labeling. This was accomplished by adding an equal volume of a citrate buffer. Citrate buffers ranging from 100 mM to 500 mM and from pH 3.4 to 5.2 were equally effective. A 200 mM citrate pH 5.0 buffer was chosen for all future studies. Previous stability studies of antimyosin Fab-DTPA in 100 mM citrate pH 5.0 indicated no loss of immunoreactivity up to 66 weeks at 5°C.

The best formulation was formulation 90, which has the composition 10 mM phosphate, 145 mM NaCl, 10% maltose and containing 0.5 mg/ml antimyosin Fab-DTPA. This formulation, at pH 7.2, was pumped into vials and compared with the previous 100 mM citrate pH 5.0 formulation. The results are shown in Table 5:

## TABLE 5

### Comparison of Citrate and Phosphate pH 7.2 Formulations

| Formulation | Conditions | Particulates | % In-III Incorp. | Immunoreactivity |
|---|---|---|---|---|
| I | 37°C shaken | | | |
| | 90 hr | +/- | 95.5 | 90.1 |
| | shipped | + | 97.6 | 90.3 |
| II | 37°C, shaken | | | |
| | 90 hr | - | 95.9 | 90.4 |
| | shipped | - | 95.1 | 90.4 |

I = 0.5 mg/mL antimyosin Fab-DTPA in 100 mM citrate pH 5.0

II = 0.5 mg/mL antimyosin Fab-DTPA in 10 mM phosphate pH 7.2, 145 mM NaCl, 10% maltose

The phosphate formulation (Formulation II) showed no particulates under these conditions, still bound In-111, and maintained a high level of immunoreactivity.

Example 2

Stress testing of Antimyosin Fab - DTPA Formulations

MATERIALS AND METHODS

Vials of antimyosin Fab-DTPA (Centocor, Inc.) were stored under the following conditions:
4°C for 12 months
ambient temperature for 12 months
37°C for 12 months
and analyzed for particulates by particle counting and visual inspection.

Two vials each of antimyosin Fab-DTPA were stored at 4°C, and then subjected to the following stresses immediately prior to analyis:
24 hours at -20°C

24 hours at 45°C

24 hours at -20°C followed by 24 hours at 45°C

shipment at ambient temperature from Malvern, PA, to Miami, FL, and back (Federal Express Priority 1) untreated control stored at 4°C.

All vials of antimyosin Fab-DTPA were filled at 1.15 ml/vial and contained 0.5 mg protein per ml of buffer solution. The buffer solution was composed of 100 mM sodium phosphate (pH 6.5), 145 mM NaCl, 10% maltose.

Visual Inspection

All vials were inspected for visible particulates prior to analysis and the results recorded on a scale of 1 to 5, with 1 meaning no visible particles and 5 meaning that numerous, easily visible particulates were present. Table 6 summarizes the results of visual inspection and particle counting after stressing.

## TABLE 6

### Visual Inspection and Particle Counting

| Treatment | Visual (1) Inspection | Particle Counting (2) | |
|---|---|---|---|
| | | 10 microns | 25 microns |
| 24 hr., -20°C | 3 | 465 | 177 |
| 24 hr, 45°C | 3 | 840 | 318 |
| 24 hr, -20°C/24 hr. 45°C | 2 | 686 | 253 |
| shipping at ambient temp. | 2 | 648 | 295 |
| storage at 4°C (control) | 1 | 625 | 226 |
| 12 mo. at 4°C | 2 | 571 | 288 |
| 12 mo. at ambient | 3 | 587 | 280 |
| 12 mo. at 37°C | 3 | 575 | 261 |

(1) Average of observations of two vials, graded on a scale of 1 to 5, with 1 meaning no visible

particles, 5 meaning numerous visible particles or fibers.

(2) Average of analysis of three 0.1 ml aliquots, and expressed as total particles per 1.15 ml (one dose).

In order to avoid diluting samples and possibly dissolving particles, three 0.1 ml aliquots were analysed, then averaged and converted to total particles per dose (1.15 ml). No differences were observed by particle counting and all samples met USP XXI requirements. However, differences were seen by visual inspection. By this subjective evaluation, the numbers of visible particles increased in all groups relative to the untreated control stored at 4°C.

Full Scale Labeling

The contents of one stressed vial of antimyosin Fab-DTPA were transferred to a vial containing 1 ml of 200 mM citrate pH 5.0 buffer, (Centocor, Inc.). Indium-111 chloride (Amersham, cat. no. INS-IPA) was diluted with expired, unopened Indium-111 chloride to a concentration of 1 mCi/ml, and 0.25 ml was added to the citrate buffered antimyosin Fab-DTPA. After incubation for 10 minutes at room temperature, protein bound Indium-111 was determined by ITLC-SG chromatography by the following method: Ten microliters were spotted 1.5 cm from one end of a 1 x 10 cm ITLC-SG paper strip (Gelman cat. no. 61885) and developed in 100 mM sodium citrate (pH 5.0). The strip was cut in half and both halves measured in a dose calibrator (Capintec CRC-5) set for In-111. Under these conditions, all In-111 that was protein bound remained at the origin. The product specification is greater than 90% protein bound at 10 minutes.

The contents were then withdrawn by syringe through an 0.2 μm filter (Millipore® Millex-GV cat no. SLGVO25LS). The syringe/filter/needle assembly and the filter/needle were weighed before and after filtration in order to measure the weight of the entire dose and the weight retained in the filter/needle. The uCi of Indium-111 in the syringe/filter/needle and in the filter/needle was measured in a dose calibrator (Capintec CRC-5) after filtration. The filtrate was then analyzed for immunoreactivity and HPLC gel filtration: Gel filtration was performed using high performance liquid chromatography (HPLC) by injecting 10 μl of each sample onto a Dupont Zorbax GF-250 (0.94 x 25 cm) column at a flow rate of 1 ml/min. The mobile phase was 200 mM sodium phosphate pH 6.8, and the eluate was monitored for absorbance at 214 nanometers. The absorbance signal was integrated, and elution time and integrated area of each peak determined. The product specification is greater than 98.0% of all protein elutes as monomer Fab-DTPA.

Full scale clinical labelings were carried out on the remaining unopened vial from each stress condition, using In-111 diluted to 1 mCi/ml. The loss of mass and radioactivity were measured to determine if the stress caused radioactivity to be preferentially held up by the filter. The results show in in Table 7, indicated that the losses range from 7.0 to 11.5% and were not substantially different from the control.

## TABLE 7

## Loss of Mass and Radioactivity on Filter Unit

| Sample Filter | % Total Dose (g) Retained by Filter | % Total In-111 Retained By |
|---|---|---|
| 24 hr., -20°C | 9.1 | 7.8 |
| 24 hr., 45°C | 9.7 | 8.4 |
| 24 hr., -20°C/24 hr. 45°C | 9.0 | 7.0 |
| shipping at ambient temp. | 11.5 | 8.9 |
| control stored at 4°C | 10.4 | 8.0 |

These losses are attributed to the expected holdup of solution in the filter. The radioactivity retained by the filter averaged 1.8% less than the percent mass retained. Each full scale labeling was also evaluated for In-111 incorporation and immunoreactivity as shown in Table 8. All of the samples were within specifications for these two lots.

## TABLE 8

### Evaluation of Full Scale Labelings

| Sample | % In-111 Protein Bound | % Immunoreactivity |
|---|---|---|
| 24 hr., -20°C | 94.6 | 95.1 |
| 24 hr., 45°C | 93.6 | 96.4 |
| 24 hr., -20°C/24 hr. 45°C | 93.0 | 98.0 |
| shipping at ambient | 94.8 | 95.4 |
| control stored at 4°C | 94.9 | 97.3 |

### Isolation of Particulates

The second stressed vial of antimyosin Fab-DTPA was opened in a laminar flow hood and analyzed on the particle counter. Each vial was opened and analyzed in a horizontal laminar flow hood. Three 0.1 ml aliquots were counted in a Climet model CI-1000 particle analyzer set to count all particles greater than or equal to 10 $\mu$m and all particles greater than or equal to 25 $\mu$m. USP XXI specifies that a single dose must contain 10,000 or fewer particles greater than or equal 10 $\mu$m in size and 1,000 or fewer particles greater than or equal to 25 $\mu$m in size. The remaining contents (about 0.5ml) were transferred to a centrifugal filter unit (Rainin cat. no 38-120 assembled with a 6 mm diameter 0.2 micron polyvinylidene difluoride membrane punched from 47 mm stock (Gelman, cat. no FP-200). The unit was centrifuged at 1000 rpm for 30 min. in a Sorvall model GLC-2B centrifuge with a HL4 rotor. Particles collected on the membrane were resuspended in 50 $\mu$l of distilled, deionized water, transferred to a microfuge tube and dissolved by vortexing. The dissolved particles were analyzed by HPLC gel filtration, SDS-PAGE chromatography and isoelectric focusing IEF as described below. The filtrate was analyzed by optical density at 280 nanometers ($OD_{280}$), HPLC gel filtration, SDS-PAGE chromatography, and IEF.

### Protein Concentration

The optical density at 280 nanometers was measured using a UV spectrophotometer (Milton Roy model 1201). An 0.1 ml aliquot of sample was diluted with 0.4 ml of buffer lacking protein, and the instrument was blanked with the same buffer. The $OD_{280}$ reading was converted to mg protein per ml assuming $E^{0.1\%} = 1.4$ for murine immunoglobulins. Product specification is 0.45-0.55 mg/ml.

### SDS PAGE

Sodium dodecyl sulfate polyacrylamide gel electrophoresis (SDS-PAGE) using gradient gels and a discontinuous buffer system was carried out on the Pharmacia PhastSystem using PhastGel gradient 10-15 precast gels and PhastGel SDS buffer strips. The 0.5 mm thick gels have a 4.5% T (total weight of acrylamide monomer and cross-linker), 3% C (amount of the crosslinker) stacking gel above a continuous 10 to 15% gradient gel (2% C) and a buffer system of 0.112 M acetate, 0.112 M Tris, pH 6.4. The SDS buffer strips contain 0.20M tricine, 0.20 M Tris, 0.55% SDS at pH 7.5 in a 2% agarose gel.

Equal volumes of sample and 2x sample buffer (5% w/v sodium dodecyl sulfate, 0.02% bromophenol blue, with or without 10% v/v 2-mercaptoethanol) were heated for 5 minutes in a boiling water bath and 1 ul of each applied to a sample lane. Low molecular weight markers (Bio-Rad cat. no. 161-0304) were included on each gel. The gels were run for 60-65 Vhr with the limiting conditions of 250V, 10 mA and 3.0 W.

The gels were stained in the PhastSystem development unit first with Coomassie Blue, then with silver nitrate using the PhastGel silver stain kit (Pharmacia). Gels were stained for 8 min. in. 0.1% w/v Phast Gel Blue R in 30% v/v methanol, 10% v/v acetic acid for 5 min., then switched to fresh destain for 8 min., then switched again to fresh destain for 10 min. Gels were preserved in 5% v/v glycerol, 10 v/v acetic acid for 5 min. Each gel was then washed for 2 min. in 10% v/v ethanol, 5% v/v acetic acid, then fresh wash solution

EP 0 417 191 B1

added and washed for an additional 4 min. (50°C). The gel was treated with 5% v/v glutaraldehyde for 6 min. at 50°C, followed by two washes with 10% v/v ethanol, 5% v/v acetic acid for 3 min. and 5 min., then two washes with distilled water for 2 min. each, all at 50°C. Gels were stained with 0.4% w/v silver nitrate for 6.5 min. at 40°C, then washed twice in distilled water each for 0.5 min. at 30°C. Developer (2.5% w/v sodium carbonate, 0.013% v/v formaldehyde) was added twice for 0.5 min. and 4 min. at 30°C. The gels were treated to reduce background for 2 min. at 30°C with 2.5% w.v sodium thiosulfate, 3.7% w/v Tris-HCL. The gel was preserved by washing for 5 min. at 50°C in 5% v/v glycerol. Gels were photographed and allowed to air dry.

The product specification is that the sample must conform to standard, in this case, the untreated control.

## Isoelectric Focusing

Isoelectric focusing (IEF) was performed using Pharmacia PhastGel IEF 3-9 precast gels of range 3 to 9 pH units. The gels are approximately 0.5mm thick homogeneous polyacrylamide gels (5% T, 3%C) containing Pharmalyte carrier ampholytes. The gels were prefocused for 75 Vhr using the limiting conditions of 2000V, 2.5 mA and 3.5 W. The gels were then run for 410 Vhr using the same limiting conditions as during prefocusing. IEF gels were stained in the PhastSystem development module as described under SDS-PAGE. The product specification is that the sample must conform to standard, in this case the untreated control.

## Column Immunoreactivity Assay

Aliquots of the In-111 labeled antimyosin used to determine In-111 incorporation were also used to measure the immunoreactivity. At the same time that the ITLC-SG chromatography was performed, a 1000-fold dilution of the radiolabeled antimyosin Fab-DTPA was made in 0.01 M sodium phosphate pH 7.2, 150 mM naCl, 1%(w/v) bovine serum albumin (PBS-1% BSA). One hundred $\mu$l of this diluted sample was applied to a 1 ml column of myosin-Sepharose® C1-4B. This affinity column was prepared by the attachment of myosin purified from dog heart tissue to cyanogen-bromide activated Sepharose®C1-4B (Pharmacia). The column was eluted with ten 1 ml aliquots of PBS-1% BSA, followed by ten 1 mL aliquots of 0.1 M glycine pH 2.5, 0.01% thimerosal. The collected fractions were counted in a gamma counter (LKB model 1272) set for In-111, the percentage eluting with the glycine buffer representing active radiolabeled antibody. This percentage was divided by the fraction of In-111 protein bound (from ITLC-SG chromatography) to correct for unbound In-111. The product specification is greater than 85% immunoreactivity.

The antimyosin Fab-DTPA remaining in each vial after particle counting (0.5 ml) was filtered by centrifugation through an 0.2 micron polyvinylidene difluoride filter. This method was chosen in order to allow evaluation of both the filtrate and particulates.

Both the filtrate and the redissolved particulates were compared by HPLC gel filtration, SDS-PAGE and IEF. No aggregates could be seen in any of the gel filtration profiles. Essentially no Fab-DTPA could be detected in any of the redissolved particulates. Similarly, all of the treated samples appeared equivalent to the untreated control in both the SDS-PAGE and IEF analyses. No bands were present in the lanes containing the redissolved particulates.

The $OD_{280}$ of the filtrate was measured after 5-fold dilution, then converted to mg/ml. No significant differences from the control sample were observed. The results are shown in Table 9.

## TABLE 9

### Protein Concentration After Filtration

| Sample | OD$_{280}$ | Protein (1) Concentration (mg/ml) |
|---|---|---|
| 24 hr., -20°C | 0.113 | 0.40 |
| 24 hr., 45°C | 0.122 | 0.44 |
| 24 hr., -20°C/24 hr 45°C | 0.121 | 0.43 |
| shipping at ambient | 0.116 | 0.41 |
| storage at 4°C (control) | 0.114 | 0.41 |

(1) $\dfrac{OD280}{1.4} \times 5 = mg/ml$

Although slight increases in the number of visible particles were observed after vials were stressed, changes were not detectable by any other means of evaluation. When the particulates and filtrate were separated, no aggregates could be seen in the HPLC gel filtration profile. The redissolved particulates did not contain detectable amounts of Fab-DTPA by HPLC gel filtration, SDS-PAGE or IEF. The protein concentration measurements of the filtrates were all similar to the control, and all were below specification (0.45-0.55 mg/ml).

The stress conditions also had no effect on the results of the full scale labelings. In-111 incorporation and immunoreactivity were within specifications.

Example 3

Antifibrin Fab-DPTA Formulation

Using the same analytical techniques described in Example 1, formulation development was also performed using a second murine monoclonal antibody Fab fragment, antifibrin, conjugated to the metal chelator DTPA. The formulations which showed the best results were based on sodium phosphate, sodium chloride and maltose, and had a pH in the range of 6.0 to 7.2.

Antifibrin Fab-DTPA (Centocor, Inc.) was formulated at a protein concentration of 0.5 mg/ml into buffer solutions containing 10 mM sodium phosphate, 145 mM sodium chloride and 10% w/v maltose at pH values of 7.20, 7.00, 6.50, 6.25, 6.00 by equilibrium dialysis as described in Example 1, and vialed at 0.8 ml per vial. These vials were shipped at ambient temperature and visually inspected. The results, shown in Table 10, indicate that the formation of particulates in this formulation appeared to be pH dependent.

## TABLE 10
### Shipping Study of Antifibrin Formulation[a]

| pH | Particulates |
|------|------|
| 7.20 | + |
| 7.00 | + |
| 6.75 | +/- |
| 6.50 | +/- |
| 6.25 | - |
| 6.00 | - |

**(a) 10 mM sodium phosphate, 145 mM sodium chloride, 10% w/v maltose**

The best formulations were at pH 6.00 and 6.25, which showed no visible particles. These lower pH vials were also analyzed for In-III incorporation, and shown to be equivalent to standard untreated antifibrin Fab-DTPA.

After storage at 4°C for 2 years, the shipped vials were analyzed by particle counting as described in Example 2. The results are summarized in Table 11.

## TABLE 11
### Particle Counts for Antifibrin Formulation[a]
### Shipped and Stored at 4°C for 25 Months

| pH | total particles per dose 10 µm | 25 µm |
|------|------|------|
| 7.20 | 1810 | 910 |
| 7.00 | not tested | not tested |
| 6.75 | 280 | 170 |
| 6.50 | 600 | 360 |
| 6.25 | 470 | 250 |
| 6.00 | 840 | 430 |
| specifications[b] | 10,000 | 1,000 |

(a) 10 mM sodium phosphate, 145 mM sodium chloride, 10% w/v maltose

(b) USP XXI

These particle counts comport with the shipping study results, indicating that the higher pH formulations are less stable. The data indicate that the formulation comprising 10 mM sodium phosphate, 145 mM sodium chloride, 10% w/v maltose, when optimized for pH (e.g., pH 6 to 6.75), prevented protein preciptiation from stress induced by shipping and long term storage (> 2 years).

17

EP 0 417 191 B1

**Claims**

1. An aqueous buffer solution for monoclonal antibodies or monoclonal antibody fragments, comprising:
   (a) a phosphate buffer;
   (b) sodium chloride;
   (c) maltose; and
   (d) monoclonal antibodies or monoclonal antibody fragments.

2. An aqueous buffer of Claim 1 wherein the phosphate buffer comprises sodium phosphate having a concentration between 10 mM and 100 mM and having a pH between 6-8.

3. An aqueous buffer of claim 2 which comprises between 5 to 20 percent weight per volume maltose, for example 10 percent.

4. An aqueous buffer of Claim 1 wherein the monoclonal antibodies are antimyosin antibody molecules, or fragments.

5. An aqueous buffer of Claim 4 wherein the monoclonal antibodies are conjugated with a chelating agent and, for example, the chelating agent is DTPA.

6. An aqueous buffer of Claim 1 wherein the monoclonal antibodies are antifibrin antibody molecules, or fragments, which optionally are conjugated with a chelating agent, for example DTPA.

7. An improved aqueous solution of Claim 1 containing antimyosin or antifibrin monoclonal antibodies, containing sodium phosphate buffer and sodium chloride, wherein the improvement comprises incorporating between 5 to 20 percent by weight maltose into the solution whereby the stability of the antibody in solution is enhanced by the presence of the maltose.

8. An aqueous buffer solution for monoclonal antibodies, which comprisies:
   (a) 10 mM to 100 mM sodium phosphate, with a pH between 6-8;
   (b) 145 mM sodium chloride;
   (c) 5-10% weight per volume maltose; and
   (d) 0.5 to about 5.2 mg/ml of a monoclonal antibody Fab fragment that has been conjugated with a metal chelator, for example DTPA.

9. An aqueous buffer solution of Claim 8 wherein the antibody is specific for cardiac myosin or fibrin.

10. An aqueous buffer solution of Claim 8 which comprises:
    (a) 10 mM sodium phosphate;
    (b) 145 mM sodium chloride;
    (c) 10% weight per volume maltose, and
    (d) 0.5 mg/ml Fab-DTPA specific for cardiac myosin.

11. An aqueous buffer solution of Claim 10 wherein the sodium phosphate has a pH of 6.0-6.75.

12. An aqueous buffer solution of claim 8 which comprises:
    (a) 10 mM sodium phosphate;
    (b) 145 mM sodium chloride;
    (c) 10% weight per volume maltose, and
    (d) 0.5 mg/ml Fab-DTPA specific for fibrin.

13. An aqueous buffer solution of Claim 10, 11 or 12 wherein the sodium phopsphate has a pH of between 6.0-6.25.

**Patentansprüche**

1. Wäßrige Pufferlösung für monoklonale Antikörper oder monoklonale Antikörper-Fragmente, wobei die Pufferlösung aufweist:
   (a) einen Phosphatpuffer;

(b) Natriumchlorid;
(c) Maltose; und
(d) monoklonale Antikörper oder monoklonale Antikörper-Fragmente.

2. Wäßrige Pufferlösung nach Anspruch 1, wobei die Phosphatpufferlösung Natriumphosphat aufweist, das eine Konzentration zwischen 10 mM und 100 mM und einen pH-Wert zwischen 6 und 8 hat.

3. Wäßrige Pufferlösung nach Anspruch 2, die zwischen 5 und 20 % (Gew./Vol.), beispielsweise 10 %, Maltose aufweist.

4. Wäßrige Pufferlösung nach Anspruch 1, wobei die monoklonalen Antikörper Antimyosin-Antikörpermoleküle oder -fragmente sind.

5. Wäßrige Pufferlösung nach Anspruch 4, wobei die monoklonalen Antikörper mit einem Chelatbildner konjugiert sind und der Chelatbildner beispielsweise DTPA ist.

6. Wäßrige Pufferlösung nach Anspruch 1, wobei die monoklonalen Antikörper Antifibrin-Antikörpermoleküle oder -fragmente sind, die fakultativ mit einem Chelatbildner, beispielsweise DTPA, konjugiert sind.

7. Verbesserte wäßrige Lösung nach Anspruch 1, die monoklonale Antimyosin- oder Antifibrin-Antikörper enthält und die Natriumphosphatpuffer und Natriumchlorid enthält, wobei die Verbesserung folgendes aufweist: Einbringen von zwischen 5 und 20 Gew.-% Maltose in die Lösung, wobei die Stabilität des Antikörpers in Lösung durch die Anwesenheit der Maltose erhöht wird.

8. Wäßrige Pufferlösung für monoklonale Antikörper, die aufweist:
(a) 10 mM bis 100 mM Natriumphosphat mit einem pH-Wert zwischen 6 und 8;
(b) 145 mM Natriumchlorid;
(c) 5-10 % (Gew./Vol.) Maltose; und
(d) 0,5 bis ca. 5,2 mg/ml eines monoklonalen Antikörper-Fab-Fragments, das mit einem Metallchelator, beispielsweise DTPA, konjugiert ist.

9. Wäßrige Pufferlösung nach Anspruch 8, wobei der Antikörper für Cardio-Myosin oder -Fibrin spezifisch ist.

10. Wäßrige Pufferlösung nach Anspruch 8, die aufweist:
(a) 10 mM Natriumphosphat;
(b) 145 mM Natriumchlorid;
(c) 10 % (Gew./Vol.) Maltose; und
(d) 0,5 mg/ml Fab-DTPA, das für Cardio-Myosin spezifisch ist.

11. Wäßrige Pufferlösung nach Anspruch 10, wobei das Natriumphosphat einen pH-Wert von 6,0-6,75 hat.

12. Wäßrige Pufferlösung nach Anspruch 8, die aufweist:
(a) 10 mM Natriumphosphat;
(b) 145 mM Natriumchlorid;
(c) 10 % (Gew./Vol.) Maltose; und
(d) 0,5 mg/ml Fab-DTPA, das für Fibrin spezifisch ist.

13. Wäßrige Pufferlösung nach Anspruch 10, 11 oder 12, wobei das Natriumphosphat einen pH-Wert zwischen 6,0 und 6,25 hat.

**Revendications**

1. Solution tampon aqueuse pour anticorps monoclonaux ou fragments d'anticorps monoclonaux, comprenant:
(a) un tampon de phosphate;
(b) du chlorure de sodium;
(c) du maltose; et
(d) des anticorps monoclonaux ou des fragments d'anticorps monoclonaux.

**2.** Solution tampon aqueuse selon la revendication 1, dans laquelle le tampon de phosphate comprend du phosphate de sodium dans une concentration comprise entre 10 mM et 100 mM et a un pH compris entre 6 et 8.

**3.** Solution tampon aqueuse selon la revendication 2, comprenant entre 5 et 20% en poids par volume de maltose, par exemple 10%.

**4.** Solution tampon aqueuse selon la revendication 1, dans laquelle les anticorps monoclonaux sont des molécules d'anticorps antimyosine ou des fragments de celles-ci.

**5.** Solution tampon aqueuse selon la revendication 4, dans laquelle les anticorps monoclonaux sont combinés à un agent chélateur, l'agent chélateur étant par exemple le DTPA (acide diéthylénetriamine pentacétique).

**6.** Solution tampon aqueuse selon la revendication 1, dans laquelle les anticorps monoclonaux sont des molécules d'anticorps antifibrine ou des fragments de celles-ci, qui sont éventuellement conjugués à un agent chélateur, par exemple le DTPA.

**7.** Solution aqueuse améliorée selon la revendication 1, contenant des anticorps monoclonaux antimyosine ou antifibrine, un tampon de phosphate de sodium et du chlorure de sodium, dans laquelle l'amélioration consiste à incorporer entre 5 et 20% en poids de maltose dans la solution, ce qui fait que la stabilité de l'anticorps en solution est accrue par la présence du maltose.

**8.** Solution tampon aqueuse pour anticorps monoclonaux comprenant:
(a) 10 mM à 100 mM de phosphate de sodium, avec un pH compris entre 6 et 8;
(b) 145 mM de chlorure de sodium;
(c) 5 à 10% en poids par volume de maltose; et
(d) 0,5 à environ 5,2 mg/ml d'un fragment de liaison à l'antigène (Fab) d'anticorps monoclonal qui a été conjugué à un chélateur de métal, par exemple le DTPA.

**9.** Solution tampon aqueuse selon la revendication 8, dans laquelle l'anticorps est spécifique de la myosine cardiaque ou de la fibrine.

**10.** Solution tampon aqueuse selon la revendication 8, comprenant:
(a) 10 mM de phosphate de sodium;
(b) 145 mM de chlorure de sodium;
(c) 10% en poids par volume de maltose; et
(d) 0,5 mg/ml de Fab-DTPA spécifique de la myosine cardiaque.

**11.** Solution tampon aqueuse selon la revendication 10, dans laquelle le phosphate de sodium a un pH de 6,0-6,75.

**12.** Solution tampon aqueuse selon la revendication 8, comprenant:
(a) 10 mM de phosphate de sodium;
(b) 145 mM de chlorure de sodium;
(c) 10% en poids par volume de maltose; et
(d) 0,5 mg/ml de Fab-DTPA spécifique de la fibrine.

**13.** Solution tampon aqueuse selon l'une quelconque des revendication 10 à 12, dans laquelle le phosphate de sodium a un pH compris entre 6,0 et 6,25.